# FASCICULE DE BREVET EUROPEEN

(11) **EP 0 647 255 B1**
(45) Date de publication et mention de la délivrance du brevet: **15.12.1999**
(21) Numéro de dépôt: 94914441.4
(22) Date de dépôt: 26.04.1994
(51) Int. Cl.: C09B 67/54, A61K 7/00, A61K 7/42, A61K 7/13

(54) **PROCEDE DE PREPARATION D'UN PIGMENT MELANIQUE DE FAIBLE GRANULOMETRIE ET SON UTILISATION EN COSMETIQUE**
HERSTELLUNGSVERFAHREN EINES MELANINPIGMENTS VON GERINGER KORNGRÖSSE SOWIE SEINE ANWENDUNG IN DER KOSMETIK
PROCESS FOR THE PREPARATION OF A MELANIN PIGMENT WITH A SMALL GRAIN SIZE AND ITS USE COSMETICS

(30) Priorité: 27.04.1993 FR 9304960
(43) Date de publication de la demande: 12.04.1995
(73) Titulaire: L'OREAL, 75008 Paris (FR)
(72) Inventeur: GIACOMONI, Paolo, F-95880 Enghien-les-Bains (FR); MARROT, Laurent, F-93190 Livry-Gargan (FR); MELLUL, Myriam, F-L'Hay-les-Roses (FR); COLETTE, Annick, F-94240 L'Hay-les-Roses (FR)
(74) Mandataire: Casalonga, Axel
(86) Numéro de dépôt international: FR9400467
(87) Numéro de publication internationale: WO9425531

(56) Documents cités:
- EP-A- 0 441 689
- EP-A- 0 518 773
- EP-A- 0 524 904
- WO-A-90/01919
- US-A- 4 806 344
- CHEMICAL ABSTRACTS, vol. 109, no. 4, 25 Juillet 1988, Columbus, Ohio, US; abstract no. 24238d, U. KOCH ET. AL. 'Manufacture of color-stable C.I. Disperse Yellow 23' page 60 ;colonne 1 ; & DD,A,251 358 (U. KOCH ET. AL.)
- PATENT ABSTRACTS OF JAPAN vol. 16, no. 485 (C-993)(5528) 8 Octobre 1992 & JP,A,04 175 377 (TAENAKA KOUGIYOU K. K.) 23 Juin 1992

## Description

La invention a pour objet un procédé de préparation d'un nouveau pigment mélanique de faible granulométrie, son utilisation en cosmétique, ainsi qu'une composition cosmétique le contenant et un procédé de coloration des fibres kératiniques (cheveux, poils) ou de la peau ou de protection de l'épiderme mettant en oeuvre un tel pigment.

Les pigments mélaniques sont des pigments connus en eux-mêmes. Il s'agit plus particulièrement des pigments qui sont à l'origine de la coloration des cheveux, de la peau ou des poils d'origine humaine ou animale. Ils peuvent également être préparés par synthèse, en particulier par oxydation de dérivés indoliques tels que plus particulièrement le 5,6-dihydroxyindole.

Les pigments mélaniques ayant une faible granulométrie sont particulièrement intéressants dans la mesure où ils présentent un bon pouvoir couvrant, ce qui permet de les utiliser dans des concentrations moindres comparativement aux pigments ayant une granulométrie plus élevée, en vue d'obtenir la même coloration.

L'intérêt des pigments ayant une faible granulométrie réside également dans leurs caractéristiques cosmétiques, notamment en ce qui concerne le toucher. Les compositions à base de pigments de faible granulométrie ont en effet un toucher plus doux.

Les pigments mélaniques connus ont une granulométrie généralement comprise entre 100 et 150 microns et doivent être broyés par exemple au mortier, par un broyeur, par micronisation ou par d'autres techniques de broyage, afin d'obtenir des particules ayant une granulométrie comprise entre 15 et 20 microns.

Pour obtenir une granulométrie plus faible, il est encore nécessaire de broyer ces pigments, ce qui s'effectue généralement en milieux aqueux. On constate cependant à la suite de ce broyage et même après séchage du pigment, une réagglomération des différentes particules.

Les compositions cosmétiques contenant des pigments présentés de cette façon ont souvent des caractéristiques peu cosmétiques, dans la mesure où les particules sont relativement grosses et irrégulières. Du fait de l'agglomération, les compositions sont inesthétiques, souvent peu stables et peu couvrantes.

Le document EP-A-0 518 773 concerne une composition cosmétique comprenant un mélange de nanopigments d'oxyde métallique et de pigments mélaniques dérivés de source naturelle ou synthétique de diamètre moyen compris entre 10 et 20.000 nanomètres. La distribution granulométrique de ces pigments mélaniques est toutefois insuffisante.

Les brevets US 4 806 344 et US 5 006 331 décrivent un procédé de préparation de pigments mélaniques et leur utilisation dans des compositions solaires. Ce procédé consiste tout d'abord à solubiliser de la mélanine dans de la triéthanolamine puis à la précipiter par oxydation en présence de chlorure ferrique. Toutefois, les pigments ainsi obtenus forment des agglomérats qui doivent être réduits aux moyens d'ultrasons.

Pour certaines utilisations cosmétiques, un diamètre compris entre 15 et 20 µm est excessif et le problème se pose donc de pouvoir générer des poudres de granulométrie inférieure, sans toutefois avoir recours à des étapes supplémentaires de réduction des pigments mélaniques obtenus, à la granulométrie désirée.

La demanderesse vient de découvrir un nouveau procédé de préparation de pigments mélaniques de très faible granulométrie tels que 100% en nombre des particules obtenues possède une granulométrie inférieure à 1 µm et tels qu'une population d'au moins 60% en nombre de l'ensemble des particules possède une granulométrie comprise entre 0,5 à 1,5 fois la granulométrie moyenne en nombre de cette population de particules. Le pigment obtenu est stable dans le temps, ne se réagglomérant pas. Selon ce procédé, le pigment peut être précipité directement sur des cheveux pour les colorer dans des conditions douces ou bien il peut être, après lyophilisation, incorporé dans des compositions cosmétiques. Ces compositions sont particulièrement onctueuses et lisses, de couleurs plus intenses et plus couvrantes.

Le premier objet de l'invention est donc un procédé de préparation d'un pigment mélanique dont les particules possèdent une distribution granulométrique telle que définie plus haut consistant à solubiliser une mélanine d'origine naturelle ou synthétique dans une solution aqueuse contenant au moins un agent alcalinisant et/ou contenant au moins un agent séquestrant et à précipiter la mélanine ainsi solubilisée par addition de sels de métaux alcalino-terreux.

Un deuxième objet de l'invention est un pigment mélanique tel que celui obtenu par le procédé également objet de l'invention possédant une distribution granulométrique particulière.

L'invention a également pour objet l'utilisation de ce pigment pour la coloration des cheveux ou pour la préparation de compositions cosmétiques mettant en oeuvre un tel pigment.

Un autre objet de l'invention est un procédé de coloration des fibres kératiniques mettant en oeuvre un tel pigment.

Enfin, l'invention a également pour objet un procédé de maquillage des matières kératiniques, un procédé de protection de l'épiderme humain contre les effets néfastes du rayonnement UV, mettant en oeuvre les compositions contenant au moins une dispersion de pigment mélanique de faible granulométrie tel que défini ci-après.

D'autres objets de l'invention apparaîtront à la lecture de la description et des exemples qui suivent.

Le procédé de préparation du pigment mélanique de très faible granulométrie conforme à l'invention consiste :
- à solubiliser à une température comprise entre 10°C et 50°C une mélanine naturelle et/ou synthétique dans un milieu aqueux contenant au moins un agent alcalinisant et/ou au moins un agent séquestrant, puis
- à précipiter la mélanine naturelle ou synthétique solubilisée par addition d'au moins un sel de métal alcalino-terreux.

Le précipité de pigments mélaniques de faible granulométrie ainsi obtenu peut ensuite être isolé par différentes méthodes, telles que la filtration, la centrifugation. Le pigment ainsi isolé est lyophilisé.

L'analyse granulométrique du pigment mélanique ainsi obtenu révèle que le pigment est caractérisé par une distribution granulométrique telle que 100% en nombre des particules a une granulométrie inférieure à 1 µm. En outre, l'analyse montre également une population d'au moins 60% en nombre de l'ensemble des particules ainsi préparé a une granulométrie comprise entre au moins 0,5 et au plus 1,5 fois la granulométrie moyenne en nombre de cette population de particules.

On utilise des agents alcalinisants classiques capables d'amener le pH à une valeur supérieure à 11. A titre d'exemple, on peut citer la soude et la potasse. Ces agents alcalins sont de préférence employés à des concentrations variant entre 0,5 mM et 2 M.

Les agents séquestrants utilisés conformément à l'invention, encore appelés chélateurs, sont choisis parmi les composés ayant la faculté de se combiner avec des ions bi- ou tri-valents tels que le calcium, le magnésium, le cuivre, le plomb, le fer et le chrome en formant des complexes particulièrement stables. Parmi ce type de composés on utilise de préférence l'acide éthylènediamine tétracétique (E.D.T.A.), l'acide éthylèneglycol tétracétique (E.G.T.A.), l'acide diéthylènetriaminopentacétique, l'acide éthylènediamine tétraméthylènephosphonique et leurs sels de sodium, ou bien encore l'histidine ou les citrates. Ces agents séquestrants sont généralement employés à des concentrations variant entre 0,1 mM et 3 M.

Lorsque l'on utilise de tels agents séquestrants, le pH du milieu est soit ajusté à une valeur de pH basique avec les agents alcalinisants ci-dessus soit tamponné à l'aide d'une solution tampon telle que par exemple le mélange tri-hydroxyméthylaminométhane/acide chlorhydrique.

Les sels de métaux alcalino-terreux sont choisis parmi les sels de magnésium, de calcium, de strontium, de baryum ou de radium. Selon l'invention, le chlorure de magnésium et le chlorure de calcium sont particulièrement préférés. Ces sels de métaux alcalino-terreux sont généralement employés selon l'invention à des concentrations variant entre 0,5 mM et 1 M.

Les pigments mélaniques utilisés, conformément à l'invention, comme produits de départ, peuvent être d'origine naturelle ou synthétique.

Les pigments synthétiques sont en particulier des pigments résultants de la polymérisation oxydante d'un composé indolique répondant à la formule : 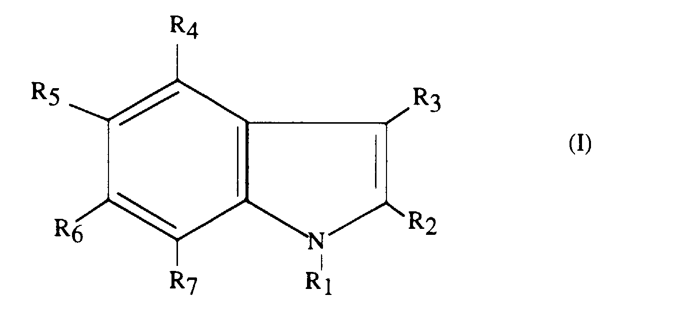 dans laquelle :
R₁ et R₃ représentent, indépendamment l'un de l'autre, un atome d'hydrogène ou un groupe alkyle en C₁-C₄;
R₂ représente un atome d'hydrogène, un groupe alkyle en C₁-C₄, un groupe carboxyle ou un groupe alcoxy(C₁-C₄)carbonyle;
R₄ et R₇ représentent, indépendamment l'un de l'autre, un atome d'hydrogène, un groupe hydroxy, alkyle en C₁-C₄, amino, alcoxy en C₁-C₄, un groupe acyl(C₂-C₄)oxy ou un groupe acyl(C₂-C₄)amino.
R₅ représente un atome d'hydrogène, un groupe hydroxy, un groupe alcoxy en C₁-C₄, un groupe alkyle en C₁-C₄, un atome d'halogène, un groupe amino, un groupe acyl(C₂-C₁₄)oxy, un groupe acyl(C₂-C₄)amino, un groupe triméthylsilyloxy ou un groupe benzyloxy;
R₆ représente un atome d'hydrogène, un groupe hydroxy, un groupe alcoxy en C₁-C₄, un groupe amino, un groupe acyl(C₂-C₁₄)oxy, un groupe acyl(C₂-C₄)amino, un groupe triméthylsilyloxy, un groupe hydroxyalkyl(C₂-C₄)amino ou un groupe benzyloxy;
R₅ et R₆ pouvant également former, conjointement avec les atomes de carbone auxquels ils sont rattachés, un cycle carbonyldioxy;
au moins l'un des radicaux R₄ à R₇ représente un groupement OZ ou NHR₈, le radical Z du groupement OZ désignant un atome d'hydrogène, un groupe acyle en C₂-C₁₄, un groupe alkyle en C₁-C₄, un groupe triméthylsilyle ou un groupe benzyle, le radical R₈ du groupement NHR₈ désignant un atome d'hydrogène, un groupe acyle en C₂-C₄ ou hydroxyalkyle en C₂-C₄, l'un au plus des radicaux R₄ à R₇ représentant NHR₈ et deux au plus des radicaux R₄ à R₇ représentant OZ, et dans le cas où Z représente un atome d'hydrogène, les deux groupes OH sont dans les positions 5 et 6; et au moins l'un des radicaux R₄ à R₇ représente un atome d'hydrogène, et dans le cas où un seul de ces radicaux R₄ à R₇ représente un atome d'hydrogène, alors un seul radical parmi R₄ à R₇ représente NHR₈ ou OZ, les autres radicaux représentant un groupe alkyle en C₁-C₄;
et leurs sels de métaux alcalins, alcalino-terreux, d'ammonium ou d'amines.

Les composés indoliques de formule (I) ci-dessus, sont choisis parmi le 4-hydroxyindole, le 5-hydroxyindole, le 6-hydroxyindole, le 7-hydroxyindole, le 4-hydroxy 5-méthoxyindole, le 4-hydroxy 5-éthoxyindole, le 2-carboxy 5-hydroxyindole, le 5-hydroxy 6-méthoxyindole, le 6-hydroxy 7-méthoxyindole, le 5-méthoxy 6-hydroxyindole, le 5,6-dihydroxyindole, le N-méthyl 5,6-dihydroxyindole, le 2-méthyl 5-6-dihydroxyindole, le 3-méthyl 5,6-dihydroxyindole, le 2,3-diméthyl 5,6-dihydroxyindole, le 2-carboxy 5,6-dihydroxyindole, le 4-hydroxy 5-méthylindole, le 2-carboxy 6-hydroxyindole, le 6-hydroxy N-méthylindole, le 2-éthoxycarbonyl 5,6-dihydroxyindole, le 4-hydroxy 7-méthoxy 2,3-diméthylindole, le 4-hydroxy 5-éthoxy N-méthylindole, le 6-hydroxy 5-méthoxy 2-méthylindole, le 6-hydroxy 5-méthoxy 2,3-diméthylindole, le 6-hydroxy 2-éthoxycarbonylindole, le 7-hydroxy 3-méthylindole, le 5-hydroxy 6-méthoxy 2,3-diméthylindole, le 5-hydroxy 3-méthylindole, le 5-acétoxy 6-hydroxyindole, le 5-hydroxy 2-éthoxycarbonylindole, le 6-hydroxy 2-carboxy 5-méthylindole, le 6-hydroxy 2-éthoxycarbonyl 5-méthoxyindole, le 6-N,β-hydroxyéthylaminoindole, le 4-aminoindole, le 5-aminoindole, le 6-aminoindole, le 7-aminoindole, le N-méthyl 6-β-hydroxyéthylaminoindole, le 6-amino 2,3-diméthylindole, le 6-amino 2,3,4,5-tétraméthylindole, le 6-amino2,3,4-triméthylindole, le 6-amino 2,3,5-triméthylindole, le 6-amino 2,3,6-triméthylindole, le 5,6-diacétoxyindole, le 5-méthoxy 6-acétoxyindole, le 5,6-triméthylsilyloxyindole, l'ester phosphorique du 5,6-dihydroxyindole, le 5,6-dibenzyloxyindole, et les sels d'addition de ces composés.

Les composés indoliques particulièrement préférés sont : le 5,6-dihydroxyindole, le 6-hydroxyindole, le 7-hydroxyindole, le bromhydrate de 2-méthyl 5,6-dihydroxyindole, le 7-aminoindole, le 3-méthyl 5,6-dihydroxyindole, le 4-hydroxy 5-méthoxyindole, le 2,3-diméthyl 5-méthoxy 6-hydroxyindole.

La polymérisation oxydante des composés de formule (I) peut s'effectuer en milieu aqueux, eau/solvant(s), ou solvant(s) à l'air, en présence ou non d'un agent alcalin et/ou d'un agent oxydant tels que le peroxyde d'hydrogène, de préférence en présence d'un agent alcalin tel que l'ammoniaque ou en présence d'ions iodure, l'iodure étant de préférence un iodure de métal alcalin, alcalino-terreux ou d'ammonium.

L'oxydation du composé de formule (I) peut également être effectué en utilisant l'acide périodique et ses sels hydrosolubles et dérivés, les permanganates et bichromates, tels que de sodium ou de potassium, l'hypochlorite de sodium, le persulfate d'ammonium, le nitrite de sodium et des oxydants organiques choisis parmi les ortho-et parabenzoquinones, les ortho- et parabenzoquinones mono- ou diimines, les 1,2- et 1,4-naphtoquinones, les 1,2- et 1,4-naphtoquinones mono ou diimines, telles que décrites dans la demande EP-A-0 376 776. Le sel d'acide périodique préféré est le périodate de sodium. n est possible d'activer les agents oxydants par un modificateur de pH.

Le procédé de polymérisation oxydante préféré met en oeuvre le peroxyde d'hydrogène en présence d'ammoniaque. Cette réaction d'oxydation s'effectue généralement à une température de l'ordre de 20°C à 100°C et de préférence 60° C à 90°C.

n est également possible de procéder à la formation des pigments mélaniques synthétiques de départ conformes à l'invention par oxydation par voie enzymatique. Cette oxydation s'effectue dans un milieu oxydant en présence d'une enzyme à activité oxydante ou peroxydante telle que les enzymes choisies parmi la peroxydase de raifort, la chloroperoxydase, la peroxydase du lait, la cytochrome C-peroxydase, ainsi que des produits ayant une activité similaire à celle des enzymes peroxydantes telles que l'hémoglobine, la methémoglobine, la myoglobine, la metmyoglobine. Cette oxydation enzymatique peut également s'effectuer en présence de tyrosinase avec l'oxygène de l'air. En particulier, le pigment indolique peut être obtenu par polymérisation de la tyrosine en présence de tyrosinase avec l'oxygène de l'air.

La réalisation de la polymérisation oxydante s'effectue de préférence en introduitsant le composé indolique de formule (I) dans un milieu aqueux, ou dans un mélange d'eau et d'un ou plusieurs solvants pouvant contenir jusqu'à 95 % de solvant ou encore dans un ou plusieurs solvants anhydre(s) c'est-à-dire contenant moins de 1 % d'eau.

Parmi les solvants utilisables, on peut citer les alcools inférieurs en C₁-C₄ tels que l'alcool éthylique, l'alcool propylique ou isopropylique, l'alcool tert.-butylique, les alkylèneglycols tels que l'éthylèneglycol, le propylèneglycol, les alkyléthers d'alkylèneglycols tels que les éthers monométhylique, monoéthylique ou monobutylique de l'éthylène glycol, les monométhyléthers de propylèneglycol et du dipropylèneglycol, et des esters tels que le lactate de méthyle. Le milieu solvant préféré est un milieu hydroalcoolique contenant de 1 à 10 % d'alcool éthylique.

Suivant les procédés, on laisse l'oxydant et le composé indolique de formule (I) en contact pendant quelques minutes à quelques jours.

Les agents alcalinisants sont de préférence choisis parmi l'hydroxyde de sodium, les carbonates alcalins ou l'ammoniaque, dans des proportions comprises entre 5 x 10⁻⁴ % à 10 % en poids par rapport au poids de la composition soumise à l'oxydation.

Lorsqu'on utilise un iodure en présence de peroxyde d'hydrogène, on utilise de préférence l'iodure de sodium ou de potassium à une concentration comprise entre 1 et 6 %.

Le pigment coloré résultant de la polymérisation oxydante est obtenu sous forme insoluble. Il est isolé par filtration ou centrifugation. Afin d'éliminer les traces de composé de formule (I) n'ayant pas réagi, on peut rincer le pigment à l'eau avant ou après filtration ou centrifugation.

Dans le cas où l'on met en oeuvre un procédé de polymérisation oxydante à l'air, il est également possible d'isoler le pigment par lyophilisation.

On obtient ainsi un pigment mélanique ayant une granulométrie de 100-150 µm qui et alors broyé par voie classique comme par exemple par broyeur au mortier, par micronisation ou d'autres techniques de broyage, jusqu'à ce que sa granulométrie soit de l'ordre de 15 à 20 µm.

A titre de mélanine naturelle, on peut par exemple utiliser de la mélanine extraite de fibres kératiniques telles que les cheveux humains ou encore de la mélanine de seiche.

Ces pigments mélaniques sont employés dans le procédé de l'invention à des concentrations variant entre 0,1 et 10 %.

Après mise en oeuvre du procédé de l'invention on obtient un pigment mélanique caractérisé par une distribution granulométrique telle que plus de 100% en nombre des particules a une granulométrie inférieure à 1 µm. En outre, une population d'au moins 60% en nombre de l'ensemble des particules a une granulométrie comprise entre au moins 0,5 et au plus 1,5 fois la granulométrie moyenne en nombre de cette population de particules. Ces pigments mélaniques constituent également un objet de 1' invention.

L'invention a également pour objet l'utilisation de ces pigments mélaniques de faible granulométrie pour la préparation de compositions cosmétiques ou la coloration des cheveux.

Le pigment mélanique de très faible granulométrie obtenu selon le procédé de l'invention peut être utilisé, après lyophilisation, pour la préparation de compositions cosmétiques.

Dans ces compositions, la concentration en pigments mélaniques selon l'invention est comprise entre 0,001 et 20 % en poids.

La dilution du pigment mélanique de l'invention n'en modifie pas sa qualité en ce sens que la granulométrie reste sensiblement constante sans qu'il y ait de réagglomération et/ou de sédimentation.

Lorsque les compositions sont utilisées pour le maquillage de la peau, des cils et des sourcils, tel que crème de traitement de l'épiderme, fond de teint, bâton de rouge à lèvre, fard à paupières, fard à joues, ligneur encore appelé "eye-liner", ou mascara, elles peuvent se présenter sous forme solide ou pâteuse, anhydre ou aqueuse, comme des émulsions huile-dans-eau ou eau-dans-l'huile, ou encore des dispersions, des lotions plus ou moins épaissies, des sticks ou des poudres.

Lorsque les compositons sont utilisées pour le maquillage des ongles, elles peuvent se présenter sous forme aqueuse ou anhydre.

Ces compositions présentent l'avantage d'être particulièrement stables et de présenter une bonne inocuité.

Lorsque les compositions sont utilisées pour la protection de l'épiderme humain contre le rayonnement UV, elles constituent des compositions dites "solaires" et elles se présentent généralement sous forme de dispersions dans des solvants ou des corps gras, ou encore sous forme d'émulsions telles que crèmes et laits, de pommades, de gels, de bâtonnets solides ou de mousses aérosols. Les émulsions peuvent contenir en outre des agents tensio-actifs bien connus dans l'état de la technique, tels que des agents tensio-actifs anioniques, non-ioniques, cationiques ou amphotères.

Lorsque la composition cosmétique selon l'invention est utilisée pour la coloration des cheveux, elle peut se présenter sous forme de shampooing, de lotion, de gel ou composition à rincer, à appliquer avant ou après shampooing, avant, pendant ou après permanente ou défrisage, de lotion ou gel coiffants ou traitants, de lotion ou gel pour le brushing ou la mise en plis, de laque pour cheveux, de composition de permanente ou de défrisage, de coloration des cheveux.

Les compositions de maquillage et les compositions solaires peuvent également contenir des corps gras, des solvants organiques, des silicones, des épaississants, des adoucissants, des filtres solaires, des agents anti-mousses, des agents hydratants, des parfums, des conservateurs, des agents anti-oxydants, des charges, des séquestrants, des agents de traitement tels que des polymères anioniques, cationiques, non-ioniques, amphotères ou leurs mélanges, des propulseurs, des agents alcalinisants ou acidifiants.

Les corps gras peuvent être constitués par une huile ou une cire ou leur mélange, les acides gras, les alcools gras, la vaseline, la parafine, la lanoline, la lanoline hydrogénée, la lanoline acétylée.

Les huiles sont en particulier choisies parmi les huiles animales, végétales, minérales ou de synthèse et notamment l'huile de palme hydrogénée, l'huile de ricin hydrogénée, l'huile de vaseline, l'huile de paraffine, l'huile de Purcellin.

Les cires sont choisies notamment parmi les cires animales, fossiles, végétales, minérales ou de synthèse et on peut citer les cires d'abeilles, les cires de Carnauba, de Candellila, de canne à sucre, du Japon, les ozokérites, la cire de Montan, les cires microcristallines, les paraffines, les cires de silicone.

Les compositions conformes à l'invention peuvent également contenir en plus des pigments mélaniques, d'autres pigments généralement utilisés en cosmétique, notamment des pigments nacrés et/ou nacrants permettant de varier les colorations susceptibles d'être obtenues ou d'augmenter la protection vis-à-vis du rayonnement ultraviolet. Dans ce dernier cas, on utile des pigments métalliques tels que les oxydes de titane, de zinc, de cérium ou de zirconium.

On utilise préférentiellement des "nanopigments", de granulométrie inférieure à 100 nm et de préférence comprise entre 5 et 50 nm. Les nanopigments peuvent être enrobés ou non enrobés.

Un autre objet de l'invention est un procédé de coloration des fibres kératiniques et plus particulièrement des cheveux humains, qui consiste à précipiter le pigment mélanique de faible granulométrie directement sur lesdites fibres, après que celles ci aient été mises en contact avec la mélanine solubilisée, dans un milieu aqueux contenant au moins un agent alcalinisant et/ou au moins un agent séquestrant, tels que définis précédemment, par application sur ces fibres d'une composition contenant au moins un sel d'un métal alcalino-terreux tel que défini précédemment.

Les exemples suivants sont destinés à illustrer l'invention sans pour autant présenter un caractère limitatif.

### EXEMPLES DE PREPARATION

### Exemple 1

On solubilise 0,2 g de piment mélanique résultant de la polymérisation oxydative du 5,6-dihydroxyindole dans 100 g d'une solution de soude 0,1 N, sous agitation à 37°C pendant 24 heures. On ajoute à cette solution 0,95 g (0,1 M) de chlorure de magnésium. La solution précipite. Le précipité est récupéré par centrifugation puis lyophilisé. On obtient un pigment noir dont 100% des particules a une granulométrie inférieure à 1 µm et dont le diamètre moyen de ces particules est de 350 nm. En outre, une population de 70% de ces particules a une granulométrie comprise dans un domaine centré sur 160 nm.

### Exemple 2

On procède de la même façon que dans l'exemple 1 sauf que l'on remplace le chlorure de magnésium par 1,1 g (0,1 M) de chlorure de calcium. On obtient un pigment noir dont la granulométrie est comprise entre 500 et 900 nm.

### Exemple 3

On solubilise 1 g du pigment résultant de la polymérisation oxydative du 5,6-dihydroxyindole dans 100 ml de soude 1 N sous agitation pendant 2 jours à 45°C.

On ajoute à cette solution 0,95 g de chlorure de magnésium (0,1 M). La solution précipite. Après centrifugation et lyophilisation, on obtient un pigment noir dont 100% des particules a une granulométrie inférieure à 400nm et dont le diamètre moyen de particules est d'environ 220 nm.

En outre 60% en nombre de ces particules a une granulométrie comprise dans un domaine variant entre 200 et 370 nm et a une granulométrie moyenne en nombre de 250 nm.

### Exemple 4

On solubilise 5 mg de mélanine synthétique obtenue par polymérisation oxydative de la tyrosine par le persulfate d'ammonium dans 1 ml d'une solution d'histidine 0,1 M. Après dialyse contre une solution tamponnée trihydroxyméthylaminométhane/acide chlorhydrique 10 mM et dont le pH est de 8, la mélanine est mise en solution dans l'eau à raison de 0,5 mg/ml.

L'addition de 0,02 mg (2.10⁻³ M) de chlorure de magnésium entraîne la précipitation. Après lyophilisation, on obtient 100% de particules dont la granulométrie est inférieure à 800 nm et dont le diamètre moyen de particules est de 420 nm.
En outre, 66% des particules a une granulométrie comprise dans un domaine centré sur 230 nm.

### Exemple 5

De la mélanine de cheveux roux est extraite par incubation de 100 mg de cheveux roux pendant 3 heures dans 1 ml d'une solution de soude (50 mM) à une température de 70°C. Le surnageant est récupéré par centrifugation et son pH est ajusté à 9 avec de l'acide chlorhydrique. Après purification par incubation pendant une nuit avec 1 mg/ml de protéinase, le surnageant est précipité par addition d'acide chlorhydrique 1N, centrifugé, lavé avec de l'acide chlorhydrique dilué (5 mM) puis lyophilisé. Le lyophilisat est ensuite solubilisé dans une solution tamponnée trihydroxyméthyl-aminométhane/acide chlorhydrique (10 mM) et dont le pH est de 8. Le solution obtenue est ensuite précipitée par ajout de 0,01 mg (1.10⁻³M) de chlorure de magnésium. On obtient 100% de particules dont la granulométrie est inférieure à 300 nm et dont le diamètre moyen de particules est de 140 nm.

En outre 89% des particules a une granulométrie comprise dans un domaine centré sur 80 nm.

### EXEMPLES DE FORMULATION

### Exemple 1 : Coloration par précipitation sur les cheveux

Une mèche de cheveux décolorés est trempée dans 500 ml d'une solution d'E.D.T.A. 10 mM contenant 4 mg/ml de mélanine synthétique obtenue par polymérisation oxydative de la tyrosine par le persulfate d'ammonium. L'addition de 20 mM (0,95 g) de chlorure de magnésium provoque une coloration châtain clair de ces cheveux.

### Exemple 2

On prépare un mascara de formule suivante :
- acide stéarique 6,0 g
- stéarate de glycéryle 3,7 g
- cire d'abeille 5,5 g
- cire de carnauba 1,9 g
- cire de paraffine 7,5 g
- rosine 1,83 g
- parahydroxybenzoate de propyle 0,05 g
- pigment obtenu à l'exemple 1 0,8 g MA
- parahydroxybenzoate de méthyle 0,23 g
- hydroxyéthylcellulose 0,22 g
- triéthanolamine 3,0 g
- gomme d'acacia 5,8 g
- eau qsp 100,0 g

On obtient un mascara noir onctueux et lisse, présentant une fine dispersion du pigment au microscope optique.

### Exemple 3

On prépare un eye-liner de formule suivante :
- Lauryl sorbitan oxyéthyléné à 20 moles d'oxyde d'éthylène 1,5 g
- Propylèneglycol 5,0 g
- Alcool polyvinylique 20,0 g
- Ultramarin 20,0 g
- Pigment obtenu à l'exemple 1 5,0 g MA
- Alcool éthylique 5,0 g
- Conservateur 5,0 g
- Eau qsp 100,0 g

On obtient un mascara noir liquide et lisse, présentant une fine dispersion du pigment au microscope optique.

### Exemple 4

On prépare un fond de teint de composition suivante :
**A:**
   - Mélange de stéarate de glycéryl et d'ester de polyéthylèneglycol et d'acide stéarique vendu sous la dénomination "ARLACEL 165" par la Société I.C.I. 2,1 g
   - Lauryl sorbitan oxyéthyléné à 60 moles d'oxyde d'éthylène 0,9 g
   - Alcool cétylique 0,5 g
   - Acide stéarique 1,5 g
   - Polyisobutène hydrogéné 22,0 g
   - Parahydroxybenzoate de propyle 0,2 g
**B:**
   - Triéthanolamine 0,75 g
**C:**
   - Dioxyde de titane 5,0 g
   - Oxyde de fer 2,0 g
   - Pigment obtenu à l'exemple 1 0,2 g MA
**D:**
   - Glycérine 3,0 g
   - Parahydroxybenzoate de méthyle 0,2 g
   - Eau qsp 100,0 g
**E:**
   - Polydiméthylsiloxane cyclique 3,0 g
   - Carbopol 940 vendu par la Société GOODRICH 0,15 g
   - Gomme de xanthane 0,4 g

La phase **A** est fondue à une température comprise entre 70 et 80°C, puis on ajoute la phase **B**. On ajoute ensuite la phase **C** dans le mélange **A** + **B**, sous agitation et ce jusqu'à obtention d'une dispersion homogène. On ajoute ensuite la phase **D** sous agitation de façon à former une émulsion. La phase **E** est incorporée en dernier lieu, sous agitation et à une température de 40°C environ.

On obtient un fond de teint lisse et onctueux, présentant une fine dispersion du pigment au microscope optique.

## Revendications

1. Procédé de préparation d'un pigment mélanique de granulométrie inférieure à 1 µm, caractérisé par le fait qu'il consiste à solubiliser à une température comprise entre 10°C et 50°C une mélanine d'origine naturelle et/ou synthétique dans un milieu aqueux contenant au moins un agent alcalinisant et/ou au moins un agent séquestrant, à précipiter ensuite la mélanine ainsi solubilisée par addition d'au moins un sel de métal alcalino-terreux.

2. Procédé selon la revendication 1, caractérisé par le fait que la solution aqueuse contient au moins un agent alcalinisant capable d'amener le pH à une valeur supérieure à 11.

3. Procédé selon revendication 1 ou 2, caractérisé par le fait que les agents séquestrants sont choisis parmi l'acide éthylènediamine tétracétique, l'acide éthylène-glycoltétracétique, l'acide diéthylène triamino pentacétique, l'acide éthylène diamine tétraméthylène phosphonique ou leurs sels de sodium, l'histidine, les citrates.

4. Procédé selon l'une quelconque des revendications 1 à 3, caractérisé en ce que les agents alcalinisants sont présents en une concentration variant entre 0,5 mM et 2 M.

5. Procédé selon l'une quelconque des revendications 1 à 4, caractérisé par le fait que les agents séquestrants sont présents en une concentration variant entre 0,1 mM et 3 M.

6. Procédé selon l'une quelconque des revendications 1 à 5, caractérisé par le fait que les sels de métaux alcalino-terreux sont choisis parmi le chlorure de magnésium et le chlorure de calcium.

7. Procédé selon l'une quelconque des revendications 1 à 6, caractérisé par le fait que les sels de métaux alcalino-terreux sont employés à des concentrations variant entre 0,5 mM et 1 M.

8. Procédé selon l'une quelconque des revendications 1 à 7, caractérisé par le fait que la mélanine synthétique résulte de la polymérisation oxydante d'un composé indolique répondant à la formule : 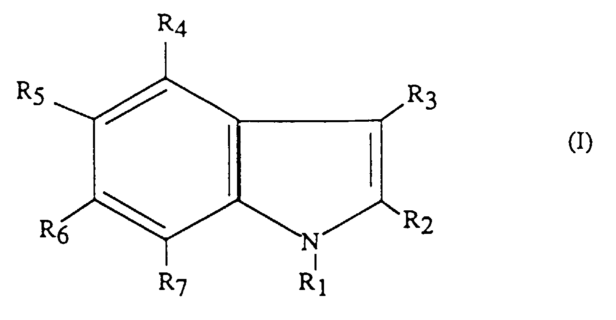 dans laquelle :
R₁ et R₃ représentent, indépendamment l'un de l'autre, un atome d'hydrogène ou un groupe alkyle en C₁-C₄;
R₂ représente un atome d'hydrogène, un groupe alkyle en C₁-C₄, un groupe carboxyle ou un groupe alcoxy (C₁-C₄)carbonyle;
R₄ et R₇ représentent, indépendamment l'un de l'autre, un atome d'hydrogène, un groupe hydroxy, un groupe alkyle en C₁-C₄, un groupe amino, un groupe alcoxy en C₁-C₄, un groupe acyl(C₂-C₄)oxy ou un groupe acyl(C₂-C₄)amino;
R₅ représente un atome d'hydrogène, un groupe hydroxy, un groupe alcoxy en C₁-C₄, un groupe alkyle en C₁-C₄, un atome d'halogène, un groupe amino, un groupe acyl(C₂-C₁₄)oxy, un groupe acyl(C₂-C₄)amino, un groupe triméthylsilyloxy ou un groupe benzyloxy;
R₆ représente un atome d'hydrogène, un groupe hydroxy, un groupe alcoxy en C₁-C₄, un groupe amino, un groupe acyl(C₂-C₁₄)oxy, un groupe acyl(C₂-C₄)amino, un groupe triméthylsilyloxy, un groupe hydroxyalkyl(C₂-C₄)amino ou un groupe benzyloxy;
R₅ et R₆ pouvant également former, conjointement avec les atomes de carbone auxquels ils sont rattachés, un cycle carbonyldioxy;
au moins l'un des radicaux R₄ à R₇ représente un groupement OZ ou NHR₈, avec le radical Z du groupement OZ désignant un atome d'hydrogène, un groupe acyle en C₂-C₁₄, un groupe alkyle en C₁-C₄, ou un groupe triméthylsilyle ou un groupe benzyle, le radical R₈ du groupement NHR₈ désignant un atome d'hydrogène, un groupe acyle en C₂-C₄ ou hydroxyalkyle en C₂-C₄, l'un au plus des radicaux R₄ à R₇ représentant NHR₈ et deux au plus des radicaux R₄ à R₇ représentant OZ et dans le cas où Z représente un atome d'hydrogène, les deux groupes OH sont dans les positions 5 et 6; et au moins l'un des radicaux R₄ à R₇ représente un atome d'hydrogène, et dans le cas où un seul de ces radicaux R₄ à R₇ représente un atome d'hydrogène, alors un seul radical parmi R₄ à R₇ représente NHR₈ ou OZ, les autres radicaux représentant un groupe alkyle en C₁-C₄;
et leurs sels de métaux alcalins, alcalino-terreux, d'ammonium ou d'amines.

9. Procédé selon l'une quelconque des revendications 1 à 7, caractérisé par le fait que la mélanine naturelle est extraite de fibres kératiniques ou encore de la mélanine de seiche.

10. Procédé selon l'une quelconque des revendications 1 à 9, caractérisé en ce que la mélanine naturelle ou synthétique est employée à une concentration variant entre 0,1 et 10 % en poids du poids total de la solution aqueuse.

11. Pigment mélanique caractérisé par une distribution granulométrique telle que 100% en nombre de ces particules a une granulométrie inférieure à 1 µm.

12. Produit constitué de particules de pigment mélanique selon la revendication 11, caractérisé par le fait qu'une population d'au moins 60% en nombre de l'ensemble des particules a une granulométrie comprise entre au moins 0,5 et au plus 1,5 fois la granulométrie moyenne en nombre de cette population de particules.

13. Utilisation du pigment mélanique de faible granulométrie obtenu par la mise en oeuvre du procédé tel que défini dans l'une quelconque des revendications 1 à 10, ou tel que défini dans la revendication 11 ou 12 pour la préparation de compositions cosmétiques.

14. Utilisation du pigment mélanique de faible granulométrie obtenu par la mise en oeuvre du procédé tel que défini dans l'une quelconque des revendications 1 à 10 pour la coloration des cheveux.

15. Composition cosmétique destinée au maquillage de la peau, des cils, des sourcils et des ongles contenant au moins un pigment mélanique de faible granulométrie obtenu par la mise en oeuvre du procédé tel que défini dans l'une quelconque des revendications 1 à 10 ou tel que défini dans la revendication 11 ou 12.

16. Composition cosmétique destinée à la protection de l'épiderme humain contre le rayonnement UV contenant au moins un pigment mélanique de faible granulométrie obtenu par la mise en oeuvre du procédé tel que défini dans l'une quelconque des revendications 1 à 10, ou tel que défini dans la revendicatin 11 ou 12.

17. Composition cosmétique selon la revendication 16 ou 17, caractérisée en ce qu'elle contient de 0,001 à 20 % en poids par rapport au poids total de la composition, de pigment mélanique de faible granulométrie obtenu par la mise en oeuvre du procédé tel que défini dans l'une quelconque des revendications 1 à 10, ou tel que défini dans la revendication 11 ou 12.

18. Procédé de teinture des fibres kératiniques et plus particulièrement des cheveux, caractérisé en ce qu'un pigment mélanique de granulométrie inférieure à 1 µm, est directement précipité sur lesdites fibres après mise en contact de ces fibres avec la mélanine solubilisée dans un milieu aqueux contenant au moins un agent alcalinisant et/ou au moins un agent séquestrant puis par application sur ces mêmes fibres d'une composition contenant au moins un sel de métal alcalino-terreux tel que défini dans l'une quelconque des revendications 6 ou 7.

## Patentansprüche

1. Verfahren zur Herstellung eines Melaninpigments mit einer Korngröße unter 1 µm, dadurch gekennzeichnet, daß es darin besteht, ein Melanin natürlichen und/oder synthetischen Ursprungs bei einer Temperatur im Bereich von 10 °C bis 50 °C in einem wässerigen Medium, das mindestens ein Mittel zum Alkalischmachen und/oder mindestens ein Maskierungsmittel enthält, zu solubilisieren und das so solubilisierte Melanin anschließend durch Zugabe mindestens eines Erdalkalimetallsalzes auszufällen.

2. Verfahren nach Anspruch 1, dadurch gekennzeichnet, daß die wässerige Lösung mindestens ein Mittel zum Alkalischmachen enthält, das befähigt ist, den pH-Wert auf einen Wert über 11 zu bringen.

3. Verfahren nach Anspruch 1 oder 2, dadurch gekennzeichnet, daß die Maskierungsmittel unter Ethylendiamintetraessigsäure, Ethylenglykoltetraessigsäure, Diethylentriaminpentaessigsäure, Ethylendiamintetramethylenphosphonsäure oder den Natriumsalzen dieser Verbindungen, Histidin und Citraten ausgewählt sind.

4. Verfahren nach einem der Ansprüche 1 bis 3, dadurch gekennzeichnet, daß die Mittel zum Alkalischmachen in einer Konzentration im Bereich von 0,5 mM bis 2 M vorliegen.

5. Verfahren nach einem der Ansprüche 1 bis 4, dadurch gekennzeichnet, daß die Maskierungsmittel in einer Konzentration im Bereich von 0,1 mM bis 3 M vorliegen.

6. Verfahren nach einem der Ansprüche 1 bis 5, dadurch gekennzeichnet, daß die Erdalkalimetallsalze unter Magnesiumchlorid und Calciumchlorid ausgewählt sind.

7. Verfahren nach einem der Ansprüche 1 bis 6, dadurch gekennzeichnet, daß die Erdalkalimetallsalze in Konzentrationen im Bereich von 0,5 mM bis 1 M verwendet werden.

8. Verfahren nach einem der Ansprüche 1 bis 7, dadurch gekennzeichnet, daß das synthetische Melanin das Ergebnis der oxidativen Polymerisation einer Indolverbindung der folgenden Formel ist: 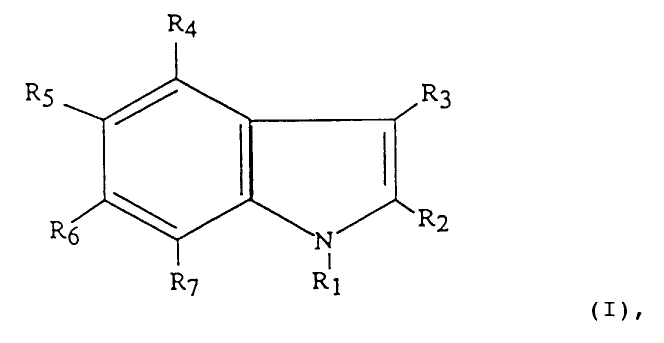 worin:
- R₁ und R₃ unabhängig voneinander ein Wasserstoffatom oder eine C₁₋₄-Alkylgruppe bedeuten,
- R₂ ein Wasserstoffatom, eine C₁₋₄-Alkylgruppe, eine Carboxygruppe oder eine C₁₋₄-Alkoxycarbonylgruppe bedeutet,
- R₄ und R₇ unabhängig voneinander ein Wasserstoffatom, eine Hydroxygruppe, eine C₁₋₄-Alkylgruppe, eine Aminogruppe, eine C₁₋₄-Alkoxygruppe, eine C₂₋₄-Acyloxygruppe oder eine C₂₋₄-Acylaminogruppe bedeuten,
- R₅ ein Wasserstoffatom, eine Hydroxygruppe, eine C₁₋₄-Alkoxygruppe, eine C₁₋₄-Alkylgruppe, ein Halogenatom, eine Aminogruppe, eine C₂₋₁₄-Acyloxygruppe, eine C₂₋₄-Acylaminogruppe, eine Trimethylsilyloxygruppe oder eine Benzyloxygruppe bedeutet,
- R₆ ein Wasserstoffatom, eine Hydroxygruppe, eine C₁₋₄-Alkoxygruppe, eine Aminogruppe, eine C₂₋₁₄-Acyloxygruppe, eine C₂₋₄-Acylaminogruppe, eine Trimethylsilyloxygruppe, eine C₂₋₄-Hydroxyalkylaminogruppe oder eine Benzyloxygruppe bedeutet,
- R₅ und R₆ ferner zusammen mit den Kohlenstoffatomen, an die sie gebunden sind, einen Carbonyldioxyring bilden können, und
- mindestens eine der Gruppen R₄ bis R₇ eine Gruppe OZ oder NHR₈ bedeutet, wobei die Gruppe Z der Gruppe OZ ein Wasserstoffatom, eine C₂₋₁₄-Acylgruppe, eine C₁₋₄-Alkylgruppe, eine Trimethylsilylgruppe oder eine Benzylgruppe und die Gruppe R₈ der Gruppe NHR₈ ein Wasserstoffatom, eine C₂₋₄-Acylgruppe oder eine C₂₋₄-Hydroxyalkylgruppe bedeuten,
wobei:
- höchstens eine der Gruppen R₄ bis R₇ NHR₈ und höchstens zwei der Gruppen R₄ bis R₇ OZ bedeuten, wobei für den Fall, daß Z ein Wasserstoffatom bedeutet, die beiden OH-Gruppen in 5-Stellung und in 6-Stellung vorliegen, und
- mindestens eine der Gruppen R₄ bis R₇ ein Wasserstoffatom bedeutet, wobei für den Fall, daß nur eine der Gruppen R₄ bis R₇ ein Wasserstoffatom bedeutet, nur eine der Gruppen R₄ bis R₇ NHR₈ oder OZ und die übrigen Gruppen eine C₁₋₄-Alkylgruppe bedeuten, und ihrer Alkalimetallsalze, Erdalkalimetallsalze, Ammoniumsalze oder Aminsalze.

9. Verfahren nach einem der Ansprüche 1 bis 7, dadurch gekennzeichnet, daß das natürliche Melanin aus Keratinfasern extrahiert ist oder daß es sich um Tintenfischmelanin handelt.

10. Verfahren nach einem der Ansprüche 1 bis 9, dadurch gekennzeichnet, daß das natürliche oder synthetische Melanin in einer Konzentration im Bereich von 0,1 bis 10 Gew.-% des Gesamtgewichts der wässerigen Lösung verwendet wird.

11. Melaninpigment, gekennzeichnet durch eine Korngrößenverteilung, die so ist, daß 100 % der Anzahl der Partikel eine Korngröße unter 1 µm aufweisen.

12. Produkt, bestehend aus Partikeln des Melaninpigments nach Anspruch 11, dadurch gekennzeichnet, daß eine Population von mindestens 60 % der Gesamtzahl der Partikel eine Korngröße aufweist, die im Bereich von mindestens dem 0,5-fachen bis höchstens dem 1,5-fachen des Zahlenmittels der Korngröße dieser Partikelpopulation liegt.

13. Verwendung des durch Anwendung des in einem der Ansprüche 1 bis 10 definierten Verfahrens hergestellten oder des in Anspruch 11 oder 12 definierten Melaninpigments mit geringer Korngröße zur Herstellung kosmetischer Zusammensetzungen.

14. Verwendung des durch Anwendung des in einem der Ansprüche 1 bis 10 definierten Verfahrens hergestellten Melaninpigments mit geringer Korngröße zum Färben der Haare.

15. Kosmetische Zusammensetzung, die zum Schminken der Haut, der Wimpern, der Augenbrauen und der Nägel bestimmt ist und die mindestens ein durch Anwendung des in einem der Ansprüche 1 bis 10 definierten Verfahrens hergestelltes oder ein in Anspruch 11 oder 12 definiertes Melaninpigment mit geringer Korngröße enthält.

16. Kosmetische Zusammensetzung, die zum Schutz der menschlichen Epidermis gegenüber UV-Strahlung bestimmt ist und die mindestens ein durch Anwendung des in einem der Ansprüche 1 bis 10 definierten Verfahrens hergestelltes oder ein in Anspruch 11 oder 12 definiertes Melaninpigment mit geringer Korngröße enthält.

17. Kosmetische Zusammensetzung nach Anspruch 15 oder 16, dadurch gekennzeichnet, daß sie bezogen auf das Gesamtgewicht der Zusammensetzung 0,001 bis 20 Gew.-% des durch Anwendung des in einem der Ansprüche 1 bis 10 definierten Verfahrens hergestellten oder des in Anspruch 11 oder 12 definierten Melaninpigments mit geringer Korngröße enthält.

18. Verfahren zum Färben von Keratinfasern und insbesondere der Haare, dadurch gekennzeichnet, daß ein Melaninpigment mit einer Korngröße unter 1 µm direkt auf den Fasern ausgefällt wird, nachdem die Fasern mit dem in einem wässerigen Medium, das mindestens ein Mittel zum Alkalischmachen und/oder mindestens ein Maskierungsmittel enthält, solubilisierten Melanin in Kontakt gebracht wurden, indem anschließend eine Zusammensetzung, die mindestens ein in einem der Ansprüche 6 oder 7 definiertes Erdalkalimetallsalz enthält, auf die Fasern aufgetragen wird.

## Claims

1. Process for the preparation of a melanic pigment of particle size smaller than 1 µm, characterized in that it consists in dissolving a melanin of natural and/or synthetic origin at a temperature of between 10°C and 50°C in an aqueous medium containing at least one alkalifying agent and/or at least one sequestering agent, and in then precipitating the melanin thus dissolved by the addition of at least one alkaline-earth metal salt.

2. Process according to Claim 1, characterized in that the aqueous solution contains at least one alkalifying agent capable of bringing the pH to a value higher than 11.

3. Process according to Claim 1, characterized in that the sequestering agents are chosen from ethylenediaminetetraacetic acid, ethylene glycol tetraacetic acid, diethylenetriaminopentaacetic acid, ethylenediaminetetramethylenephosphonic acid or their sodium salts, histidine and citrates.

4. Process according to any one of Claims 1 to 3, characterized in that the alkalifying agents are present in a concentration varying between 0.5mM and 2M.

5. Process according to any one of Claims 1 to 4, characterized in that the sequestering agents are present in a concentration varying between 0.1mM and 3M.

6. Process according to any one of Claims 1 to 5, characterized in that the salts of alkaline-earth metals are chosen from magnesium chloride and calcium chloride.

7. Process according to any one of Claims 1 to 6, characterized in that the salts of alkaline-earth metals are used in concentrations varying between 0.5mM and 1M.

8. Process according to any one of Claims 1 to 7, characterized in that the synthetic melanin results from the oxidative polymerization of an indolic compound corresponding to the formula: 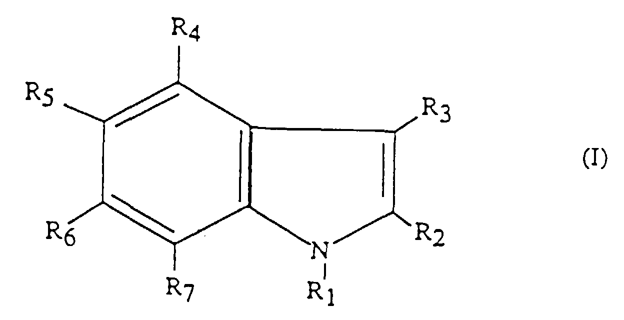 in which:
R₁ and R₃ denote, independently from one another, a hydrogen atom or a C₁-C₄ alkyl group,
R₂ denotes a hydrogen atom, a C₁-C₄ alkyl group, a carboxyl group or a (C₁-C₄ alkoxy)carbonyl group,
R₄ and R₇ denote, independently of one another, a hydrogen atom, a hydroxyl group, a C₁-C₄ alkyl group, an amino group or a C₁-C₄ alkoxy group, a (C₂-C₄ acyl)oxy group or a (C₂-C₄ acyl)amino group,
R₅ denotes a hydrogen atom, a hydroxyl group, a C₁-C₄ alkoxy group, a C₁-C₄ alkyl group, a halogen atom, an amino group, a (C₂-C₁₄ acyl)oxy group, a (C₂-C₄ acyl)amino group, a trimethylsilyloxy group or a benzyloxy group,
R6 denotes a hydrogen atom, a hydroxyl group, a C₁-C₄ alkoxy group, an amino group, a (C₂-C₁₄ acyl)oxy group, a (C₂-C₄ acyl)amino group, a trimethylsilyloxy group, a (C₂-C₄ hydroxyalkyl)amino group or a benzyloxy group,
it being also possible for R₅ and R₆ to form, jointly with the carbon atoms to which they are attached, a carbonyldioxy ring;
at least one of the radicals R₄ to R₇ denotes a group OZ or NHR₈, the radical Z of the group OZ denoting a hydrogen atom, a C₂-C₁₄ acyl group, a C₁-C₄ alkyl group, a trimethylsilyl group or a benzyl group, the radical R₈ of the group NHR₈ denoting a hydrogen atom, a C₂-C₄ acyl or C₂-C₄ hydroxyalkyl group, not more than one of the radicals R₄ to R₇ denoting NHR₈ and not more than two of the radicals R₄ to R₇ denoting OZ, and, in the case where Z denotes a hydrogen atom, the two OH groups are in positions 5 and 6; and at least one of the radicals R₄ to R₇ denotes a hydrogen atom and, in the case where only one of these radicals R₄ to R₇ denotes a hydrogen atom while a single radical from R₄ to R₇ denotes NHR₈ or OZ, the other radicals denoting a C₁-C₄ alkyl group;
and their alkali or alkaline-earth metal, ammonium or amine salts.

9. Process according to any one of Claims 1 to 7, characterized in that the natural melanin is extracted from keratinous fibres or from squid melanin.

10. Process according to any one of Claims 1 to 9, characterized in that the natural or synthetic melanin is employed in a concentration varying between 0.1 and 10 % by weight of the total weight of the aqueous solution.

11. Melanic pigment characterized by a particle size distribution such that 100 % of the number of these particles have a particle size smaller than 1 µm.

12. Product consisting of particles of melanic pigment according to Claim 11, characterized in that a population of at least 60 % of the number of all the particles has a particle size of between at least 0.5 and not more than 1.5 times the number-average particle size of this particle population.

13. Use of the melanic pigment of small particle size, obtained by implementing the process as defined in any one of Claims 1 to 10 or as defined in Claim 11 or 13, for the preparation of cosmetic compositions.

14. Use of the melanic pigment of small particle size, obtained by implementing the process as defined in any one of Claims 1 to 10 for colouring hair.

15. Cosmetic composition intended for making-up skin, eyelashes, eyebrows and nails, containing at least one melanic pigment of small particle size, obtained by implementing the process as defined in any one of Claims 1 to 10 or as defined in Claim 11 or 12.

16. Cosmetic composition intended for the protection of the human epidermis against UV radiation, containing at least one melanic pigment of small particle size, obtained by implementing the process as defined in any one of Claims 1 to 10 or as defined in Claim 11 or 12.

17. Cosmetic composition according to Claim 15 or 16 characterized in that it contains from 0.001 to 20 % by weight, relative to the total weight of the composition, of melanic pigment of small particle size, obtained by implementing the process as defined in any one of Claims 1 to 10, or as defined in Claim 11 or 12.

18. Process for dyeing keratinous fibres and more particularly hair, characterized in that a melanic pigment of particle size smaller than 1µm is precipitated directly on the said fibres after these fibres have been brought into contact with melanin dissolved in an aqueous medium containing at least one alkalifying agent and/or at least one sequestering agent and then by application to these same fibres of a composition containing at least one alkaline-earth metal salt as defined in either of Claims 6 and 7.
